# EUROPEAN PATENT APPLICATION

(11) **EP 2 759 601 A1**
(43) Date of publication of application: **30.07.2014**
(21) Application number: 12833119.6
(22) Date of filing: 20.09.2012
(51) Int. Cl.: C12Q 1/68

(54) **GENE COPY NUMBER VARIATION MEASUREMENT METHOD**

(30) Priority: 22.09.2011 CN 201110284638
(71) Applicant: Guo, Qiwei, Xiamen, Fujian 361000 (CN); Zhou, Yulin, Xiamen, Fujian 361000 (CN)
(72) Inventor: Guo, Qiwei, Xiamen, Fujian 361000 (CN); Zhou, Yulin, Xiamen, Fujian 361000 (CN)
(74) Representative: Verscht, Thomas Kurt Albert
(86) International application number: PCT/CN2012/081644
(87) International publication number: WO 2013/041031

(57) **Abstract**

Disclosed is a gene copy number variation measurement method. The method selects an endogenous or exogenous similar sequence similar, but not identical, to the gene sequence to be measured, designs the corresponding primers for the gene sequence to be measured and the similar sequence to amplify at the same time, and then performs a melting curve analysis. When the gene to be measured experiences copy number variations, the melting curve analysis result thereof exhibits substantial differences from the analysis result of the wild gene to be measured, thereby achieving the measurement objective. The method of the present invention is applicable not only to the measurement of gene copy number variations, but also applicable to the detection of chromosome aneuploid. The method of the present invention is not only applicable to conventional prenatal checkups and the screening for people having gene copy number variations and chromosome aneuploid, but also for non-invasive prenatal checkups.

## Description

### Field of the invention

The present invention relates to the detection of copy number variations (CNVs), particularly detection that combines similar sequences with melting curve analysis.

### Background of the invention

CNVs were first identified in the fruit fly *bar* gene 70 years ago and were subsequently considered to commonly occur in animal and plant genomes. Some copy number variations are regarded as polymorphisms because these do not result in a change in the phenotype of an organism. However, some CNVs truly affect gene expression by changing the order of genes or altering the gene content, thus causing phenotype variations and adaptations, as well as diseases. It is conservatively estimated that at least 10% of the human genome consists of copy number variants. If a functional gene such as *SMN* or *HER2* exists as copy number variants, this could lead to aberrant amounts of its gene product, thus causing an abnormal phenotype, which in turn lead to diseases such as spinal muscular atrophy (SMA) and breast cancer. Aneuploidy is a typical CNV phenomenon that involves the deletion or addition of an entire chromosome in a somatic cell, often leading to syndromes such as Turner's syndrome (45,X), Klinefelter's syndrome (47, XXY), and Down's syndrome (47, +21). These inherited diseases not only have a high prevalence but are also uncurable. Therefore, population screening and prenatal diagnosis are particularly important for the control of these inherited diseases.

Existing methods for the detection of CNVs include the following:
Comparative genomic hybridization (CGH): This technology has been developed as a combination of microarray technology with comparative genomic hybridization technology (array-CGH). This technology can detect the copy number of whole chromosomes or chromosomal fragments at the sub-band level. However, the resolution of this technology is a million base pairs. Therefore, smaller copy number fragments are beyond its level of detection. In addition, this technology is labor-intensive, time-consuming, and costly. It also requires large amounts of DNA for analysis, thus rendering the method impractical for extensive applications.
2. Multiplex ligation-dependent probe amplification (MLPA): MLPA is a detection method for CNVs and was developed in 2002. Commercial MLPA kits for the detection of diseases such as SMA and Turner's syndrome are currently available. This technology has been extensively applied worldwide. However, its methodology for probe preparation is relatively complex and time-consuming. Furthermore, the use of capillary electrophoresis renders this approach labor-intensive and costly and leads to a high risk for carry-over contamination.
3. High resolution melting (HRM) curve analysis: HRM was developed in 2003; it identifies polymerase chain reaction (PCR) products by estimating the melting temperature of the DNA sequence by accurately controlling the temperature ramp rate and the use of DNA saturation dyes. Because this technology is rapid, inexpensive, and has high-throughput, it has been extensively applied in research studies involving single nucleotide polymorphism (SNP), mutation, and epigenetic analyses. HRM has been applied to the detection of CNVs via SNP locus detection within target sequences. Loss of heterozygosity often occurs in CNVs and this difference is readily detected using HRM analysis. HRM curve analysis of SNPs has some limitations. First, this method requires that the SNP locus is heterozygous; otherwise, it cannot distinguish between two allelic sequences. Therefore, detecting a single SNP locus is only effective in some of the cases, and combined analysis of several SNP loci is required for more cases, which not only increases the cost and complexity of the analysis but also decreases the output of the test. Furthermore, SNP-based strategies have provided poor results in the detection of aneuploidy because the changes in the melting curves were too small to distinguish from those for normal samples.

### Summary of the invention

The objective of the present invention is to provide a method for the detection of CNVs by conducting melting curve analysis of similar sequences.

To achieve above object, the method of the technical proposal consists of the following steps:
1) screening similar endogenous reference sequences or synthetic similar exogenous reference sequences in the entire genome according to the target sequence;
2) aligning the target sequence and the reference sequence and designing common amplification primers;
3) amplifying the target sequence and the reference sequence in a same reaction tube using a pair of common primers; and
4) analyzing the PCR product by melting curve analysis.

The target sequence and the reference sequence are either paralogous or non-paralogous.

The PRR products are designed with corresponding fluorophore-labled oligonucleotide probe or non-labeled oligonucleotide probe,

The similar sequence includes a sequence converted by bisulfite.

The common amplification primer is a pair or several pairs of amplification primers used for multiple locus analysis.

The common amplification primer completely or does not completely match the template.

The melting curve analysis consists of regular melting curve analysis and high-resolution melting curve analysis.

The melting curve analysis involves the signals generated by a DNA saturation dye, a DNA saturation dye combined with non-labeled oligonucleotide probe, or a fluorophore-labeled oligonucleotide probe.

The DNA saturation dye is EvaGreen dye, LC Green^{®} PLUS dye, ResoLight dye, or SYTO 9 dye.

The fluorophore-labeled oligonucleotide probe is a hydrolysis probe, molecular beacon, opposite probe, cyclic probe, or a double-stranded probe.

Data processing pertains to the melting curve analysis, which comprises normalization, temperature shifting, and difference plotting.

The method is not only applied to the detection of CNVs but also to dosage-variant genes such as *SMN* and *HER2,* as well as cases of aneuploidy, including Turner's syndrome, Klinefelter's syndrome, and Down's syndrome.

The method has a high level of detection resolution; it is not only applicable to screening large populations and prenatal samples, but also mosaicism and cell-free fetal DNA in maternal plasma. Therefore, the present invention is applicable to noninvasive prenatal testing.

Similar sequences pertain to at least two nucleotide sequences with at least 20% sequence similarity, preferably at least 70% similarity, but optimally 90% similarity. In the case of two similar sequences, similarity pertains to two sequences with 1-30 nucleotide differences, preferably 2-20 nucleotide differences, or optimally 2-10 nucleotide differences, and the rest are identical. According to the theory, as long as there is at least one nucleotide difference, CNVs could be detected.

The sequence is a complete sequence of an individual gene or part of an individual gene sequence. It can also be a sequence linked by several genes. Therefore, the length of similar sequences varies but can always be PCR amplified.

The principle of this method is illustrated in Fig.l. There are numerous similar sequences in the genome; these sequences may consist of paraloguos genes such as the *ZFX* gene and the *ZFY* gene, or the *Dscam* gene and *Dscam3* gene. These can also consist of non-paralogous genes. Similar sequences located on the target chromosome are referred to as target sequences, and similar sequences located on the reference chromosome are called reference sequences. If the reference sequence is absent in the genome, a segment of the corresponding exogenous reference sequence can be artificially synthesized or introduced. The introduction of a endogenous reference sequence into the genome is called the internal standard method; the introduction of an artificially synthesized reference sequence or the introduction of exogenous reference sequences is called the external standard method. The composition of the nucleotides of the target gene sequence and the reference sequence are almost identical, with only a few differences. During melting analysis, the target sequence and the reference sequence interact and form a specific melting curve profile. In CNVs, the proportion of the target sequence to the reference sequence changes, which is reflected as the change in the melting curve profile. In theory, the difference between the similar sequences is larger than that of a single SNP. Therefore, melting curve analysis of similar sequences offers higher sensitivity and specificity than that of a single SNP. Unlike SNPs that may be homozygous or heterozygous, similar sequences are invariable and therefore, universal.

Because chromosome aneuploidy results in changes in copy number in the corresponding gene, the present invention can be used to detect aneuploidy by detecting the copy number of the corresponding gene.

Moreover, CNVs occur not only in the human genome but also in animal and plant genomes. For example, CNVs occur in maize (Theor Appl Genet. 2010 Jan; 120(2):355-67.). Therefore, the present invention can be used to detect CNVs in plant and animal genomes as well.

Compared to existing technologies, the present invention has the following advantages:
1) The test is easy to design and the results are accurate.
2) The test is not restricted by the heterozygosity of a SNP locus and is therefore theoretically applicable to all populations.
3) The difference between the target sequence and the reference sequence is larger than that of a single SNP, thus effectively improving the sensitivity and specificity of detection.
4) With high sensitivity and high specificity, the present invention is applicable to samples involving very small amounts of DNA, including dried blood spots, amniotic fluid, chorionic villi, cell-free fetal DNA from maternal plasma, and pollen.
5) Its closed operation prevents the occurrence of carry-over contamination of PCR products.
6) The operation is easy to setup, rapid (1 h), inexpensive, and has high-throughput.

### Brief description of the drawings

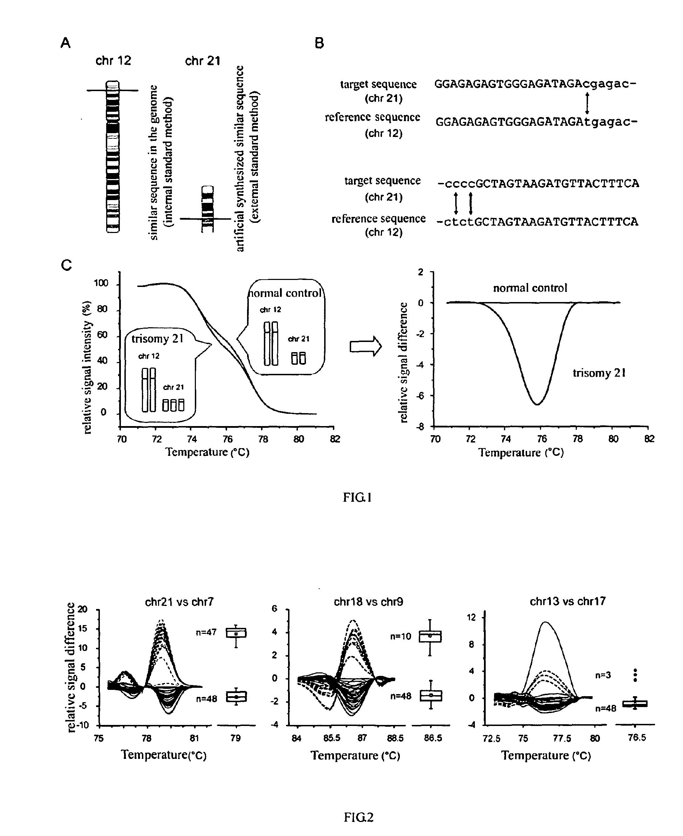

FIG.1 illustrates a schematic diagram of the present invention. (A) Taking a target sequence on chromosome 21 as an example, selecting a corresponding similar endogenous reference sequence in chromosome 12 (internal standard method). If there is no similar endogenous reference sequence, artificially synthesized or exogenous reference sequence can be used (external standard method). (B) Taking internal standard method as an example, the target sequence and the reference sequence have 3 different nucleotides in sequence, as indicated by two-way arrowhead. The capital letters indicate the primer binding locus. (C) After amplification using a single pair of primer and melting curve analysis, the trisomy 21 samples can be distinguished from the normal control samples by data analysis.
FIG.2 illustrates a detection of trisomies 13, 18, and 21 with saturation dye. The dotted lines indicate the trisomy samples, the real lines indicate the normal control samples. In statistical analysis, the upper and lower borderlines of the boxes indicate 25% and 75% confidence intervals, the dots inside the boxes indicate the mean values, the transverse lines inside the boxes indicate medians, the error bars indicate 5% and 95% confidence intervals.

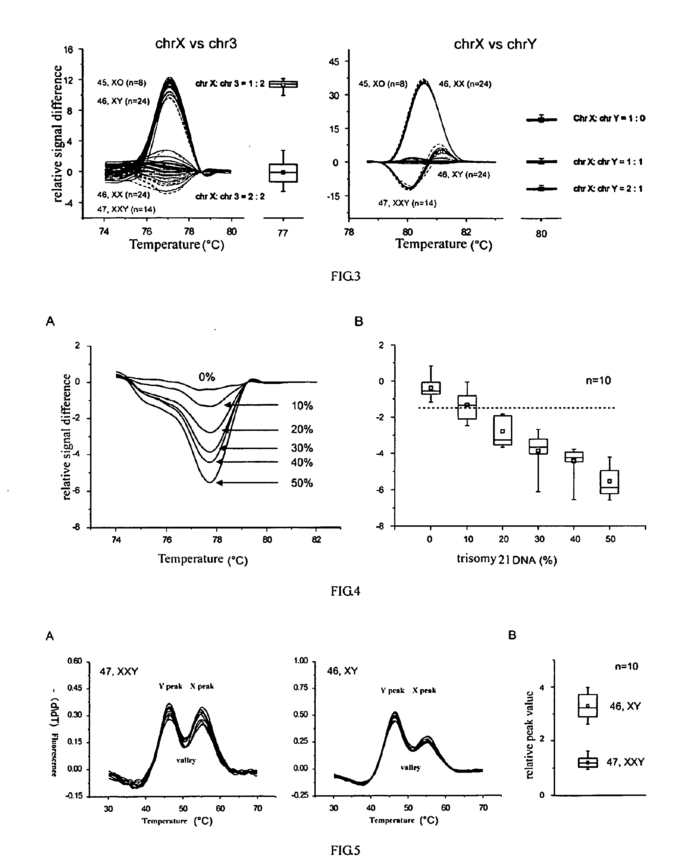

FIG.3 illustrates a detection of sex chromosome aneuploidies. The dotted lines in melting curve indicate aneuploid samples, the real lines indicate the normal control samples. In statistical analysis, the upper and lower borderlines of the boxed show 25% and 75% confidence intervals, the dots inside the boxes indicate the mean values, the transverse lines inside the boxes indicate medians, the error bars indicate 5% and 95% confidence intervals.
FIG.4 illustrates the detection of DNA mixture with different proportion of trisomy 21 DNA. (A) Melting curves of DNA mixture with different proportion of trisomy 21 DNA. Each melting curve results was plotted from 10 parallel samples. (B) In statistical analysis, the upper and lower borderlines of the boxed indicate 25% and 75% confidence intervals, the dots inside the boxes indicate the mean values, the transverse lines inside the boxes indicate medians, the error bars indicate the maximum and minimum value.
FIG.5 illustrates the detection of Klinefelter's syndrome by fluorophore-labeled oligonucleotide probe. (A) Melting curve of 10 Klinefelter's syndrome (47, XXY) samples and normal control (46, XY) samples. (B) In statistical analysis, the upper and lower borderlines of the boxes indicate 25% and 75% confidence intervals, the dots inside the boxes indicate the mean values, the transverse lines inside the boxes indicate medians, the error bars indicate 5% and 95% confidence intervals.

### Detailed description of the embodiments

The present invention will be further described with four embodiments combined with drawings, these embodiments are provided to describe some possibilities of the present invention, but not limited to the materials, conditions of the reaction or the parameters in these embodiments, any person skilled in the art can conduct the present invention of a method for the detection of CNVs by following the principle and using other similar materials or conditions of the reaction. These are not divorced from the conceptual framework of the present invention.

### The first embodiment: Detection of human autosome aneuploidies by melting analysis with saturation dye.

Trisomies 13, 18, and 21 are common human autosome aneuploidies. Trisomy 21 is also called Down's syndrome which prevalence is 1/700 in live birth. Down's syndrome has varies inherited abnormalities including learning disorder, disturbance of intelligence, deformity or other highly malformations. The disease can not be cured, and the prenatal diagnosis is an effective approach for disease control. The prevalence of trisomy 18 and 13 are 1/5000 and 1/25000, respectively. These aneuploidies also cause severe malformation and physical disability. Therefore, the prenatal diagnosis of these syndromes is also important.

### I. Materials

### 1. Instruments:

Real-time PCR system, transferpettor, centrifuge.

### 2. Primer design:

The present invention selected a similar reference sequence on chromosome 7 according to the target sequence on chromosome 21; selected a similar reference sequence on chromosome 9 according to the target sequence on chromosome 18; selected a similar reference sequence on chromosome 17 according to the target sequence on chromosome 13. 3 pairs of primer were designed to detect three aneuploidies in corresponding chromosomes (table 1), the primer sequences are:
T21-F: SEQ ID NO: 1
T21-R: SEQ ID NO: 2
T18-F: SEQ ID NO: 3
T18-R: SEQ ID NO: 4
T13-F: SEQ ID NO: 5
T13-R: SEQ ID NO: 6

### 3. Reagent

2 x LightCycler 480 high resolution melting master (Roche); Mag²⁺.

### II. Method

### 1. Sampling

Forty-eight trisomy 21 DNA samples, 10 trisomy 18 DNA samples, 3 trisomy 13 DNA samples, and 48 normal control DNA samples were tested and the results were validated with karyotyping.

### 2. PCR and HRM

PCR amplification containing 1 x LightCycler 480 high resolution melting master, 2.5 mmol/L Mg²⁺, 200 nmol/L upper stream and lower stream primers, 100 ng of genomic DNA.

PCR amplification and high resolution melting analysis were performed on a LightCycler 480 II thermocycler (Roche), the following amplification cycling conditions were used::
Stage 1: 95 °C for 10 min;
Stage 2: 95°C for 15 sec, 60°C for 15 sec, 72°C for 15 sec, for 40 cycles;
Stage 3: 95°C for 10 sec, 40°C for 10 sec, melting from 60°C to 95°C with 20 fluorescence acquisitions per s, 50°C 10 sec.

### 3. Analysis of the results

The melting curve analysis comprised normalization, temperature shifting, and difference plotting. Then the results of cases were compared to those of wild-type samples.

As illustrated in fig. 2, most of the trisomy samples and normal controls were distinctly separated from each other and segregated into different groups. The diagnostic sensitivity and specificity are 100% for the detection of trisomy 18 and 21. The diagnostic sensitivity is 100% and the diagnostic specificity is 97.9% for the detection of trisomy 13.

Table 1 the location of the similar sequences for the detection of trisomies 13, 18, and 21.

**[Table 1]**

| Primer | Location of the similar sequence (amplified sequence) |
|---|---|
| T21-F | chr21:27136956-27137019 |
| T21-R | chr7:63354651-63354714 |
| T18-F | chr18:59483387-59483477 |
| T18-R | chr9:5418670-5418760 |
| T13-F | chr13:63509758-63509823 |
| T13-R | chr17:21718218-21718283 |

### The second embodiment: Detection of human sex chromosome aneuploidies by melting analysis with saturation dye.

Except for trisomy 21, sex chromosome aneuploids such as 45, X and 47, XXY, are most prevalent in live birth. The prevalence of 45,X in female is approximately 1/2000, while the prevalence of 47,XXY in male is approximately 1/500. sex chromosome aneuploidies effects on the phenotypic development, especially in reproductive system. However, Hormone therapy can be used to improve the quality of life if sex chromosome aneuploidies are diagnosed early. Therefore, early and rapid diagnosis of sex chromosome aneuploidies is clinically important.

### I. The materials

### 1. Instruments:

real-time fluorescent PCR system, pipettor, centrifuge.

### 2. Primer design:

The present invention selected a pair of similar sequence located on chromosome 3 and X chromosome, and a pair of similar sequence located on X chromosome and Y chromosome. Two corresponding pairs of primers were listed in table 2. In theory, common sex chromosome aneuploidies can be identified by the combined analyses of these 2 types of assays (Table 3).
X3-F: SEQ ID NO: 7
X3-R: SEQ ID NO: 8
XY-F: SEQ ID NO: 9
XY-R: SEQ ID NO: 10

### 3. Reagent:

2 x LightCycler 480 high resolution melting master (Roche); Mg²⁺.

### II. The method

### 1. Samples

Eight DNA samples of Turner's syndrome (45,X), 14 DNA samples of Klinefelter's syndrome (47,XXY), 24 DNA samples of unaffected male, 24 DNA samples of unaffected female. These samples were tested and the results were validated with karyotyping.

### 2. PCR and HRM

PCR amplification containing 1 x LightCycler 480 II thermocycler (Roche) 2.5 mmol/L Mg²⁺, 200 nmol/L upper stream and lower stream primers, 100 ng of genomic DNA.

PCR amplification and high resolution melting analysis were performed on a LightCycler 480 II thermocycler, the following amplification cycling conditions were used:
Stage 1: 95 °C for 10 min;
Stage 2: 95°C for 15 sec, 60°C for 15 sec, 72°C for 15 sec, for 40 cycles;
Stage 3: 95°C for 10 sec, 40°C for 10 sec, melting from 60°C to 95°C with 20 fluorescence acquisitions per s, 50°C for 10 sec.

### 3. Analysis of the results

The melting curve analysis comprised normalization, temperature shifting, and difference plotting. Then the results of cases were compared to those of wild-type samples.

As shown in fig. 3, The samples were categorized into different groups based on their innate chromosome ratios. The assays attained 100% diagnostic sensitivity and specificity for the detection of 45,X and 47,XXY.

### Table 2

The location of the similar sequences for the detection of sex chromosome aneuploidies.

**[Table 2]**

| Designed Prime | Location of the similar sequence (amplified sequence) |
|---|---|
| X3-F | chrX:77387159-77387229 |
| X3-R | chr3:25797047-25797117 |
| XY-F | chrX:24228420-24228515 |
| XY-R | chrY:2846961-2847056 |

Table 3 Assay patterns of common sex chromosome aneuploidies.

**[Table 3]**

| Karyotype | chrX : chr3 | chrX : chrY |
|---|---|---|
| 45, X | 1:2 | 1:0 |
| 46, XX | 2:2 | 1:0 |
| 46, XY | 1:2 | 1:1 |
| 47, XXX | 2:2 | 2:1 |
| 47, XXY | 1:2 | 1:2 |
| 47, XXX | 3:2 | 1:0 |

### The third embodiment: Detection of trisomy 21 DNA in different normal DNA backgrounds.

Mosaicism is an individual with two or more than two kinds of genotype cells. According to different proportions of abnormal cells, the mosaicism exhibites a series of different degrees of symptom. In general, bigger the proportion of abnormal cells is, worse the symptom is.

Non-invasive prenatal diagnosis refers to detecting cell-free fetal DNA or RNA in the maternal plasma to obtain the genetic information of a fetus, thus avoiding the risk of miscarriage in invasive sampling. Studies have revealed that the fetal DNA fraction in first trimester is approximately 10%.

To describe the capability of the present invention in mosaicism detection and non-invasive prenatal diagnosis, we tested different ratios of trisomy 21 DNA samples and unaffected control samples.

### I. The materials

### 1. Instruments:

real-time fluorescent PCR system, pipettor, centrifuge.

### 2. Primer design:

The present invention was applied with internal standard method, using the similar sequence located on chromosome 21 and chromosome 7 as targets. The primers used are:
T21-F: SEQ ID NO: 1
T21-R: SEQ ID NO: 2

### 3. Reagent:

2 x LightCycler 480 high resolution melting master (Roche) ; Mg²⁺.

### II. The method

### 1. Samples

Ten DNA samples of trisomy 21 and 10 DNA samples of unaffected control were collected.

Mixed samples containing 10%, 20%, 30%, 40%, and 50% trisomic DNA were prepared by diluting trisomy 21 DNA with unaffected control DNA.

### 2. PCR and HRM

PCR amplification containing 1 x LightCycler 480 high resolution melting master, 2.5 mmol/L Mg²⁺ , 200 nmol/L upper stream and lower stream primers, 10 ng of genomic DNA.

PCR amplification and high resolution melting analysis were performed on a LightCycler 480 II thermocycler, the following amplification cycling conditions were used:
Stage 1: 95°C for 10 min;
Stage 2: 95°C for 15 sec, 60°C for 15 sec, 72°C for 15 sec, for 40 cycles;
Stage 3: 95°C for 10 sec, 40°C for 10 sec, melting from 60°C to 95°C with 20 fluorescence acquisitions per s, 50°C for 10 sec.

### 3. Analysis of the results

The melting curve analysis comprised normalization, temperature shifting, and difference plotting. Then the results of cases were compared to those of wild-type samples.

As shown in Fig. 4, samples containing 20% trisomic DNA were completely differentiated from the unaffected samples with no overlap. In clinical application, a mosaicism which contains more than 20% of abnormal cells can be detected. Also, if the fetal DNA fraction can be concentrated from 10% to more than 20%, the present invention can be used for noninvasive prenatal testing. The embodiment implies the capability of the present invention in mosaicism detection and non-invasive prenatal testing.

### The fourth embodiment: Detection of Klinefelter's syndrome by fluorophore-labeled probe method.

Klinefelter's syndrome (47,XXY) is the the most frequent genetic cause of non-obstructive azoospermia with the prevalence of approximately 1/500 in male. The diagnosis of 47,XXY provides the patients with an explanation about their life experiences and possible mental and physical therapies, thus providing major benefits to their quality of life Therefore, early population screening and rapid diagnosis of Klinefelter's syndrome are of importance.

### I. The materials

### 1. Instruments:

real-time fluorescent PCR system, pipettor, centrifuge.

### 2. Primer design:

Similar sequences located on X chromosome and Y chromosome are used to analyze the dosage ratio between sex chromosomes (table 2). Identical sequences of the two similar sequences are used as primer binding sites, and the different sequences of the two similar sequences are used as the probe binding sites. The detection probe XY-P is a hydrolytic probe, the 5' end is labeled with FAM , the 3' end is labeled with BHQ1. The sequence of detection probe is completely complemented with that of X chromosome, but is partially complemented with that of Y chromosome. Therefore, different melting curve profiles can be generated. The sequences of the primers and the probe are:
XY-F: SEQ ID NO: 9
XY-R: SEQ ID NO: 10
XY-P: SEQ ID NO: 11

### 3. Reagent:

1 ×PCR buffer: 10 mmol/L Tris-HCl (pH 8.3), 50 mmol/L KCl; Mg²⁺; hot start Taq DNA polymerase; dATP, dCTP, dGTP, dTTP.

### II. The method

### 1. Samples

Ten DNA samples of Klinefelter's syndrome (47,XXY), 10 DNA samples of unaffected male.

### 2. PCR and HRM

PCR amplification containing 1 x PCR buffer, 2.5nmol/L Mg²⁺, 80nmol/L of XY-F primers, 800nmol/L of XY-R primers, 400nmol/L of XY-P probe, 100 ng of genomic DNA.

PCR amplification and melting analysis were performed on a LightCycler 480 II thermocycler (Roche). The following amplification cycling conditions were used:
Stage 1: 95 °C 10 min;
Stage 2: 95°C 15 sec, 63°C 20 sec, 72°C 20 sec, for 40 cycles;
Stage 3: 95°C 10 sec, 30°C 10 sec, 30°C to 70°C collecting fluorescence 5 times per second, 50 °C 10 sec.

### 3. analysis of results

Obtaining the melting curve profiles using the the LightCycler 480 II analysis software. A typical melting cure profile is shown in fig.5A. 2 distinct peaks in the resulting melting curve were observed. Two peak values and a valley value can be generated from the melting curve. Theoretically, the relative dosage of X and Y chromosomes can be calculated using the formula: relative peak value = (Y peak value - valley value)/(X peak value - valley value), and the 47,XXY can be detected correspondingly.

As shown in fig. 5B, the relative peak values of the Klinefelter's syndrome cases are notably diffentiated from those of unaffected samples (*P*=0). Therefore, this method is robust for the detection of Klinefelter's syndrome.

### Industrial applicability

When CNVs occur in the target gene, the melting curve profiles of abnormal cases can be distinguished from those of unaffected samples using this method, thus attaining the object of detection.

## Claims

1. A method for the detection of CNVs that consists of the following steps:
Step 1, screening endogenous similar but not identical reference sequences or synthesis of exogenous similar reference sequences in the entire genome based on the target sequence;
Step 2, comparison of the target sequence and the reference sequence and designing common amplification primers;
Step 3, amplification of the target sequence and reference sequences using common primers in a reaction tube;
Step 4, analysis of the PCR products by melting curve analysis.

2. The method for the detection of CNVs as described in claim 1, wherein the corresponding fluorophore-labled oligonucleotide probe or non-labeled oligonucleotide probe can be designed based on the primer sequences.

3. The method for the detection of CNVs as described in claim 2, wherein the reference sequence and the target sequence are paralogous or non-paralogous.

4. The method for the detection of CNVs as described claim 2, wherein the reference sequence and the target sequence are located on identical chromosomes or on different chromosomes.

5. The method for the detection of CNVs as described in claim 1 or claim 2, wherein the similar sequence includes a similar sequence converted by bisulfite.

6. The method for the detection of CNVs as described in claim 1 or claim 2, wherein the common amplification primers are a pair of amplification primers or several pairs of amplification primers for multiple analysis.

7. The detection method of CNVs as described in claim 1 or claim 2, wherein the common amplification primer completely matches the template or partially matches the template.

8. The method for the detection of CNVs as described in claim 1 or claim 2, wherein the melting curve analysis comprises regular melting curve analysis and high-resolution melting curve analysis.

9. The method for the detection of CNVs as described in claim 1 or claim 2, wherein the melting curve analysis is conducted with a DNA saturation dye, a DNA saturation dye combined with non-labeled oligonucleotide probe, or a fluorophore-labeled oligonucleotide probe.

10. The method for the detection of CNVs as described in claim 1 or claim 2, wherein the DNA saturation dye includes EvaGreen dye, LC Green^{®} PLUS dye, ResoLight dye, or SYTO 9 dye.

11. The method for the detection of CNVs as described in claim 1 or claim 2, wherein the fluorophore-labeled oligonucleotide probe is a hydrolyzed probe, molecular beacon, opposite probe, cyclic probe, or double-stranded probe.

12. The method for the detection of CNVs as described in claim 1 or claim 2, wherein the data analysis of the melting curve comprise normalization, temperature shifting, and difference plotting.
